(19) Europäisches Patentamt
European Patent Office
Office européen des brevets

(11) Publication number: 0 199 325 B1

(12) # EUROPEAN PATENT SPECIFICATION

(45) Date of publication of the patent specification: 15.11.89

(51) Int. Cl.⁴: A61K 7/043

(21) Application number: 86105489.8

(22) Date of filing: 21.04.86

(54) **Silyl-containing nail enamel.**

(30) Priority: 22.04.85 US 725705

(43) Date of publication of application: 29.10.86 Bulletin 86/44

(45) Publication of the grant of the patent: 15.11.89 Bulletin 89/46

(84) Designated Contracting States: AT BE CH DE FR GB IT LI LU NL SE

(56) References cited:
DE-A- 2 428 172
DE-A- 2 804 298
DE-C- 928 728
FR-A- 1 267 528

PATENT ABSTRACTS OF JAPAN, unexamined applications, C field, vol. 9, no. 66, March, 1985 THE PATENT OFFICE JAPANESE GOVERNMENT page 92 C 271
PATENT ABSTRACTS OF JAPAN, unexamined applications, C field, vol. 5, no. 12, January 24, 1981 THE PATENT OFFICE JAPANESE GOVERNMENT page 34 C 40

(73) Proprietor: Revlon, Inc., 767 Fifth Avenue, New York, N.Y.10022(US)

(72) Inventor: Schnetzinger, Richard W., 8 Old Oak Court, Hightstown New Jersey(US)

(74) Representative: Körber, Wolfhart, Dr. et al, Patentanwälte Dipl.-Ing. H. Mitscherlich Dipl.-Ing. K. Gunschmann Dr.rer.nat. W. Körber Dipl.Ing. J. Schmidt-Evers Dipl.-Ing. W. Melzer Steinsdorfstrasse 10, D-8000 München 22(DE)

EP 0 199 325 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a new nail enamel. More particularly, it relates to nail enamels containing a siloxyanylalkyl ester and which have good stability over time, a good gloss and excellent adhesion to the nail.

Among the characteristics which nail enamels must possess include that they are not irritating towards the skin and the nails, that they give a film having satisfactory characteristics, that they are convenient to apply, that they are stable on storage, and that is to say, have a good homogeneity and a good stability with time. These characteristics of the film are essentially the production of a uniform thickness and a good gloss, which implies a smooth application surface, excellent adhesion to the nail and a satisfactory flexibility in order to prevent the enamel from breaking or crumbling.

Nail enamels are film-forming lacquers which generally are air-dried at room temperature. Nail enamel lacquers have generally comprised (1) a film former, (2) a resin, (3) a plasticizer, (4) a solvent and (5) a colorant.

Conventional nail enamels have included nitrocellulose as the film-forming constituent. Other conventional film-formers have included cellulose acetate butyrate, for example.

The resin ingredient of a nail enamel is so chosen as to modify the characteristics of the film-former employed to impart sufficient hardness, resistance to chipping, and adhesive characteristics of the dried film.

Resins known to be useful include synthetic resins such as arylsulfonamide-formaldehyde resins (e.g. toluene sulfonamide-formaldehyde resin), and various polyester and alkyd resins and naturally occurring resins such as damar. In some instances the same material may impart both the film-forming and resin hardening characteristics to nail enamel, e.g., polystyrene.

The plasticizer portion is chosen with a variety of factors being considered. The plasticizer chosen depends not only upon the composition of the film-former but upon the effect desired of the individual plasticizer on viscosity of the enamel, drying rates and the like in order to improve the adhesion and flexibility of the film.

The solvents used in nail enamel compositions are employed as the volatile portion of the lacquer. The solvent systems generally include (a) one or more active (conventional) solvents, (b) couplers (which act in effect as solvents) and (c) diluents.

The colorants incorporated in nail enamels impart the desired cosmetically acceptable shades and serve to opacify the dried films.

Accordingly, it is the general object of this invention to provide a nail enamel having good film-forming and adhesion characteristics.

Another object of the invention is to provide a nail enamel having a new film-forming constituent.

It is yet another object of this invention to provide a silyl methacrylate ester copolymer as the film-forming constituent of a nail enamel composition.

Still another object of this invention is to provide a low cost and easily handled nail enamel.

Other objects, features and advantages will be apparent from the description which follows.

In accordance with the present invention it has been found that it is possible to provide a satisfactory film-former for a nail enamel composition which dispenses with conventional film-forming agents without producing a deterioration in the properties of the film obtained by forming copolymers containing from 1 to 30% of a certain silyl methacrylate ester monomer, from 1 to 90% of certain methacrylate monomers, and 1 to 90% of certain acrylate monomers.

Typical nail enamel compositions may have solids contents varying from 20 to 35% and more, usually, from 24 to 30%. The formulations of the prior art generally contain the following:

| | |
|---|---|
| film-former | 8 to 16% |
| resin | 2 to 10% |
| plasticizer | 0.2 to 10% |
| colorant | .001 to 5% |
| solvent | balance |

The percentages given herein are (unless otherwise indicated) by weight.

The copolymers used in the nail enamels according to the invention thus make it possible to partially or totally replace the film-forming and resin components of a nail enamel formulation. This itself represents an advantage by virtue of a lower cost and of a reduction in the risks involved in handling film-forming component and formaldehyde-based resin component.

The present invention provides an anhydrous nail enamel which is characterised in that it contains a solvent system and a film former comprising a copolymer resulting from the copolymerisation of:

(1) the siloxanylalkyl ester monomer of the formula:

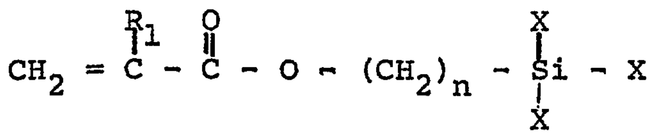

wherein each X is $OCH_3$, $OCH_2CH_3$, $OCH_2CH_2CH_3$ or a phenyl group; $R_1$ is selected from the group consisting of methyl and hydrogen; and n is an integer from three to eighteen:

(2) at least one methacrylate of the formula:

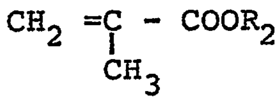 (II)

in which $R_2$ represents a linear or branched alkyl radical having from 1 to 18 carbon atoms, or the radical

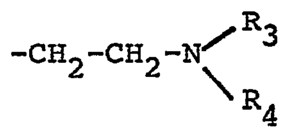

wherein $R_3$ and $R_4$ represent an alkyl or hydroxyalkyl group having from 1 to 4 carbon atoms; and

(3) at least one acrylate of the formula:

$$CH_2=CHCOOR_5 \qquad \text{(III)}$$

in which $R_5$ represents a linear or branched alkyl radical having from 1 to 18 carbon atoms or aralkyl having from 7 to 16 carbon atoms, or heteroalkyl group having from 1 to 18 carbon atoms wherein the carbon chain is interrupted by an O, S, or N.

These copolymers comprise by weight of the copolymer:

(1) at least one siloxanylakyl ester monomer of formula I, 1% to 30%, preferably 5% to 30% and more preferably 8% to 20%;

(2) at least one methacrylate monomer of formula II, 1% to 90%, preferably 5% to 85%; and

(3) at least one acrylate monomer of formula III, 1% to 90%, preferably 5% to 85%.

These copolymers are usually terpolymers but can also be tetrapolymers, pentapolymers or higher polymers insofar as more than one of the monomers from each of the three groups mentioned above is used.

Representative siloxyanylalkyl ester monomers of formula I which may be employed include trimethylsiloxanylmethyl methacrylate, trimethylsiloxanylethyl acrylate, propyl trialkoxy silyl methacrylate ester triphenyl siloxanylmethyl acrylate.

Representative methacrylate monomers of formula II which may be employed in the practice of the invention include:

methyl methacrylate
ethyl methacrylate
propyl methacrylate
isopropyl methacrylate
butyl methacrylate
amyl methacrylate
hexyl methacrylate
heptyl methacrylate
octyl methacrylate
2-ethylhexyl methacrylate
nonyl methacrylate
decyl methacrylate
undecyl methacrylate
dodecyl methacrylate
lauryl methacrylate
cetyl methacrylate
octadecyl methacrylate
oleyl methacrylate
2-N,N-dimethylamino-ethyl methacrylate

Representative acrylate monomers of formula III which may be employed in the practice of this invention include:
methyl acrylate
ethyl acrylate
butyl acrylate
2-ethyl hexyl acrylate
dodecyl acrylate
octadecyl acrylate

In another embodiment of the invention, the copolymers can also result from the copolymerization of other monomers in addition to those defined above, such as N-vinylpyrrolidone or acrylamides or methacrylamides of the formula:

$$CH_2=\underset{\displaystyle R_8}{\underset{|}{C}}-CONH-R_9 \qquad (IV)$$

in which $R_8$ represents H or $-CH_3$ and $R_9$ represents H, $-CH_2OH$

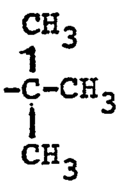 or 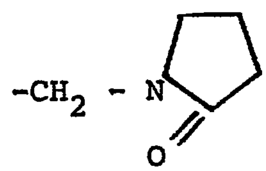

According to this embodiment, the percentage by weight of these additional monomers is suitably 1 to 20% by weight and preferably 2 to 15% by weight of the copolymer.

Finally, the following may be mentioned amongst the additional monomers corresponding to formula (IV): acrylamide methacrylamide, N-tert.-butylacrylamide, N-hydroxymethylacrylamide , N-[(N'-pyrrolidone-2)-methyl]acrylamide and n-hydroxymethylacrylamide.

The copolymers which can be used in the nail enamel compositions according to the invention can be obtained by various conventional polymerization processes, such as suspension polymerization, bulk polymerization, emulsion polymerization or solution polymerization. As the nail enamels according to the invention are to be anhydrous, the polymerization is in consequence preferably carried out in solution in an organic solvent, such as ethyl acetate, butyl acetate, acetone, or preferably ethanol.

Best results are obtained if the siloxanyl ester content of the comonomer is up to 35% by weight and correspondingly less, e.g., 10-15% with esters of higher silicon content. If one employs a branched chain acrylate ester derivative e.g., 2-ethylhexl acrylate, one preferably employs a lower siloxanyl ester comonomer, e.g., methyl siloxanylmethyl acrylate.

The copolymers of the invention are prepared by contacting the mixture of comonomers with a free radical generating polymerization initiator of the type commonly used in polymerizing ethylenically unsaturated compounds. Representative free radical polymerization initiators include:
acetyl peroxide
lauroyl peroxide
decanoyl peroxide
caprylyl peroxide
benzoyl peroxide
tertiarybutyl peroxypivalate
diisopropyl peroxycarbonate
tertiarybutyl peroctoate
N,N'-azobisisobutyronitrile

Conventional polymerization techniques can be employed to produce the novel copolymers. The comonomer mixture containing between 0.05 - 2% by weight of the free radical initiator is heated to a temperature between 30°C. - 100°C., preferably below 70°C., to initiate and complete the polymerization. The temperature is preferably maintained below 70°C. in order to minimize the formation of bubbles in the copolymer. Instead of employing the bulk polymerization techniques described above, one can employ solution, emulsion or suspension polymerization to prepare the novel copolymers, using techniques conventionally used in the preparation of polymers from ethylenically unsaturated monomers.

The copolymers used in the invention generally have a molecular weight of 2,000 to 200,000 and preferably 10,00 to 100,000, measured by osmometry.

Whether colored or colorless, the nail enamel compositions according to the present invention generally contain from 3 to 35% by weight of a copolymer as defined above, the remainder comprising the solvent system of the enamel, that is to say the customary solvents and/or conventional diluents for this type of composition.

As has been stated, the nail enamels according to the invention are anhydrous, so that the solvent system is produced from organic solvents or mixtures thereof. This makes it possible to obtain relatively short drying times. The following are included among the solvents useful in the present invention: acetone, ethyl acetate, butyl acetate, 2-methoxyethyl acetate, methyl ethyl ketone, methyl isobutyl ketone and methyl acetate. The solvent system may also include a diluent, preferably an aromatic orangic solvent, such as toluene or xylene, in a proportion which is generally from 10 to 30%, relative to the total weight of the nail enamel composition. The enamels can also contain other volatile solvents, such as ethanol, n-butanol n-propanol, isopropanol or mixtures thereof.

The nail enamels may also contain from, say, 0.2 to 10% by weight of at least one plasticizer to improve the adhesion and the flexibility of the film-forming copolymer. The following may be mentioned among the plasticizers: benzyl benzoate, tributyl phosphate, butyl acetylricinoleate, glyceryl acetylricinoleate, dibutyl phthalate, butyl glycolate, dioctyl phthalate, butyl stearate, tributoxyethyl phosphate, triphenyl phosphate, triethyl citrate, tributyl citrate, tributyl acetylcitrate, 2-triethylhexyl acetylcitrate, dibutyl tartrate, dimethoxyethyl phthalate, diisobutyl phthalate, diamyl phthalate, camphor and various mixtures thereof.

Although, with the nail enamels according to the invention, it is possible to dispense with using nitrocellulose and/or a resin of the arylsulfonamide/formaldehyde type, it is possible to introduce them into the formulations, albeit in a substantially lower proportion than that which is commonly employed. The nitrocelluloses are typically the "RS" or "SS" type, in particular 1/4 second RS type nitrocellulose, 1/2 second RS type nitrocellulose and RS 1/4 second type nitrocellulose.

The resins of the arylsulfonamide/formaldehyde type are those known by the tradenames "Santolite MHP" and "Santolite MS 30%, the former being the harder, the latter leaning to the formation of films of greater flexibility.

If the nail enamel according to the present invention is to contain a colorant at least one organic or inorganic colorant may be used. The following may be mentioned among the organic colorants: D and C Red Nos. 10, 11, 12, and 13, D and C Red No. 7, D and C Red Nos. 5 and 6, D and C Red No. 34 and lakes, such as the lake D and C YELLOW No. 5 and the lake D and C Red inorganic colorants: titanium dioxide, bismuth oxychloride, brown iron oxide, red iron oxide, and also guanine.

These colorants are preferably present in the nail enamel in an amount of 0.1 to 8% by weight, relative to the total weight of the nail enamel composition. The nail enamel compositions according to the invention can also contain other ingredients, such as products which make it possible to avoid sedimentation, and especially clays of the montmorillonite type such as bentonite, and/or a swelling agent, such as orthophosphoric acid.

The following examples further illustrate the present invention and the preparation of the copolymers used in the nail enamel compositions according to the invention.

EXAMPLE I

This example illustrates the synthesis of a representative copolymer made from a representative siloxanylalkyl ester monomer of formula I and a methacrylic ester of formula II.

A mixture of 40 grams of a propyl trialkoxy silyl methacrylate monomer, 40 grams 2-N,N-dimethylaminoethyl methacrylate, 20 grams of ethyl acrylate and 0.004 ml. of tert-butyl peroxypivalate per ml of monomer mixture are placed in a suitable flask fitted with a mechanical stirrer, a condenser and a nitrogen inlet tube.

After stirring, the reaction mixture is heated under reflux for 16 hours. After cooling, the solution is diluted with 100 g of ethyl acetate and the polymer is then precipitated by adding 7 liters of petroleum ether. After drying, the expected polymer is obtained.

EXAMPLE 2

A colorless nail enamel according to the invention may be prepared by mixing the following ingredients:
Polymer of Example 1 - 25 g
Dibutyl phthalate - 3 g
Camphor - 2 g
Ethyl acetate - 30 g
Butyl acetate - 40 g

On applying this nail enamel composition to the nails with a brush, a uniform layer is obtained which, after drying, has good adhesion and an excellent gloss.

EXAMPLE 3

A colored nail enamel is prepared according to the invention by mixing the following ingredients: Polymer of Example 1 - 25 g

Butyl phthalate - 2 g
Ethyl acetate - 20 g
Toluene - 10 g
Bentonite 27 - 1.5 g
Phosphoric acid - 0.02
Titanium oxide - 0.75
Brown iron oxide - 0.25 g
D and C RED 7-calcium lake - 0.5 g
D and C. RED 34 - 0.3 g
D and C YELLOW 5-aluminum lake - 0.7 g
Butyl acetate q.s.p. -100 g

## Claims

1. A nail enamel composition comprising a film-former and an organic solvent system, said film-former comprising a copolymer resulting from the polymerization of:

(1) 1 to 30% by weight of at least one siloxanylkyl ester monomer of the formula:

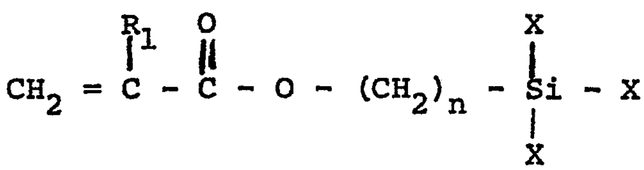

(I)

wherein each X is $OCH_3$, $OCH_2CH_3$, $OCH_2CH_2CH_3$ or a phenyl group; $R_1$ is methyl or hydrogen; and n is an integer from 3 to 18;

(2) 1 to 90% by weight of at least one methacrylate monomer of the formula:

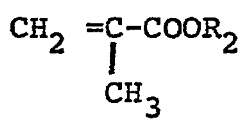

(II)

wherein $R_2$ represents a linear or branched alkyl radical having up to 18 carbon atoms, or the radical

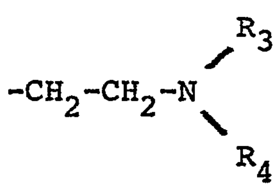

wherein $R_3$ and $R_4$ represent an alkyl or hydroxyalkyl group having from 1 to 4 carbon atoms; and

(3) 1 - 90% by weight of at least one acrylate monomer of the formula:

$$CH_2=CH-COOR_5 \qquad \text{(III)}$$

wherein $R_5$ represents a linear or branched alkyl radical having up to 18 carbon atoms, or an aralkyl radical having from 7 to 16 carbon atoms, or an heteroalkyl group having from 1 to 18 carbon atoms wherein the carbon chain is interrupted by an 0, N or S atom.

2. The nail enamel according to claim 1 in which said copolymer comprises:
(1) 5 to 20% by weight of at least one siloxanylalkyl ester monomer of formula (I),
(2) 5 to 85% by weight of at least one methacrylate monomer of formula (II), and
(3) 5 to 85% by weight of at least one acrylate monomer of formula (III).

3. The nail enamel according to claim 1 or 2 in which said copolymer also contains at least one other monomer which is N-vinylpyrrolidone or an acrylamide or methacrylamide of the formula:

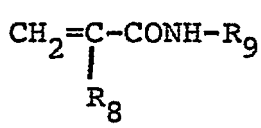

(IV)

in which $R_8$ represents H or - $CH_3$ and $R_9$ represents H, -$CH_2OH$,

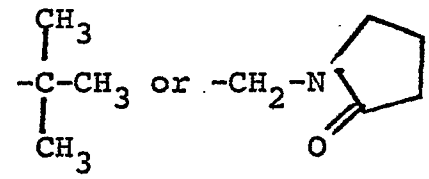

or

in an amount of 1 to 20% by weight relative to the total weight of the monomers.

4. The nail enamel according to claim 3 in which the said other monomer comprises from 2 to 15% by weight relative to the total weight of the monomers.

5. The nail enamel according to claim 3 or 4 in which the acrylamide or methacrylamide of formula (IV) is acrylamide, methacrylamide, N-tert.-butylacrylamide, N-hydroxyethylmethacrylamide , N-[(N'-pyrrolidone-2)-methyl]acrylamide or n-hydroxymethylacrylamide.

6. The nail enamel according to any of claims 1to5 in which unsaturated monomer of formula (I) is a propyl trialkoxy silyl methacrylate ester.

7. The nail enamel according to any of claims 1to6 in which the methacrylate monomer of formula (II) is methyl, ethyl, butyl, tert-butyl, hexyl, dodecyl or octadecyl methacrylates or 2-N,N-dimethylamino-ethyl methacrylate.

8. The nail enamel according to claim I in which the acrylate of formula (III) is of methyl,ethyl,butyl, 2-ethyl hexyl, dodecyl or octadecyl acrylate.

9. The nail enamel according to any of claims 1to8 in which the copolymer is quaternized with a quaternizing agent selected from dimethyl sulfate or ethyl bromide

10. The nail enamel composition according to any of claims 1to9 where the organic solvent system is acetone, ethyl acetate, butyl acetate, methyl ethyl ketone, methyl acetate or mixtures thereof.

11. The nail enamel composition according to any of claims 1to10 further comprising a plasticizer.

12. The nail enamel composition according to claim 11 wherein the plasticizer is benzyl benzoate or tributyl phosphate.

13. The nail enamel composition according to any claims 1 to 12 further comprising a colorant.

**Patentansprüche**

1. Nagellack-Zusammensetzung enthaltend einen Filmbildner und ein Lösungsmittelsystem, wobei der Filmbildner ein Copolymer umfaßt, das aus der Polymerisation von

(1) 1 bis 30 Gew.-% von wenigstens einem monomeren Siloxanylalkylester der Formel

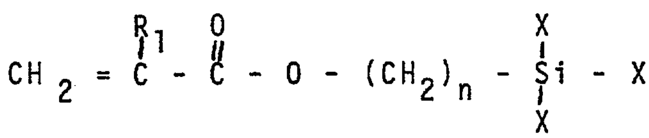

(I)

in der jedes X $OCH_3$, $OCH_2CH_3$, $OCH_2CH_2CH_3$ oder eine Phenylgruppe darstellt, $R_1$ Methyl oder H bedeutet und n eine ganze Zahl von 3 bis 18 ist,

(2) 1 bis 90 Gew.-% von wenigstens einem Methacrylat-Monomer der Formel

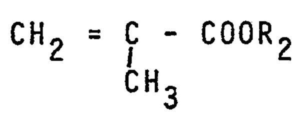

(II)

in der $R_2$ einem linearen oder verzweigten Alkylrest mit 1 bis 18 C-Atomen oder dem Rest

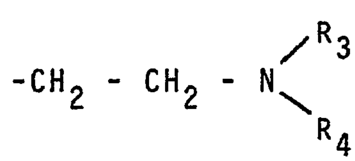

entspricht, in dem $R_3$ und $R_4$ eine Alkyl- oder Hydroxyalkylgruppe mit 1 bis 4 C-Atomen bedeutet, und

(3) 1 bis 90 Gew.-% von wenigstens einem Acrylat-Monomer der Formel

$$CH_2 = CH - COOR_5 \qquad (III)$$

in der $R_5$ einen linearen oder verzweigten Alkylrest mit bis zu 18 C-Atomen oder einem Aralkylrest mit 7 bis 16 C-Atomen oder eine durch O, N oder S unterbrochene Heteroalkylgruppe mit 1 bis 18 C-Atomen bedeutet, erhältlich ist.

2. Nagellack gemäß Patentanspruch 1, worin das Copolymer umfaßt:
(1) 5 bis 20 Gew.-% von wenigstens einem Siloxanylalkylester-Monomer entsprechend der Formel (I),
(2) 5 bis 85 Gew.-% von wenigstens einem Methacrylat-Monomer entsprechend der Formel (II) und
(3) 5 bis 85 Gew.-% von wenigstens einem Acrylat-Monomer der Formel (III).

3. Nagellack gemäß Patentanspruch 1 oder 2, in dem das Copolymer zusätzlich ein weiteres Monomer, das N-Vinylpyrrolidon oder ein Acrylamid oder Methacrylamid der Formel

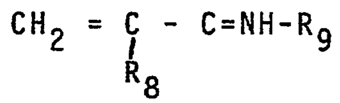
(IV)

ist, wobei $R_8$ H oder $-CH_3$ bedeutet und $R_9$ H, $-CH_2OH$

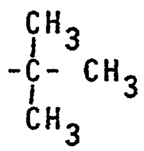 oder 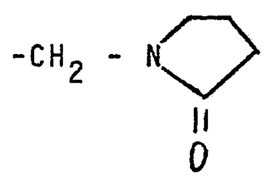

darstellt,
in einer Menge von 1 bis 20 Gew.-%, bezogen auf das Gesamtgewicht der Monomeren, enthält.

4. Nagellack gemäß Patentanspruch 3, in dem das weitere Monomer in einer Menge von 2 bis 15 Gew.-%, bezogen auf das Gesamtgewicht der Monomeren vorliegt.

5. Nagellack gemäß Patentanspruch 3 oder 4, in dem das Acrylamid oder Methacrylamid entsprechend der Formel (IV) Acrylamid, Methacrylamid, N-tert.-Butylacrylamid, N-Hydroxyethylmethacrylamid, N-(N'-Pyrrolidon-2)-methylacrylamid oder n-Hydroxymethylacrylamid ist.

6. Nagellack gemäß einem jeden der Patentansprüche 1 bis 5, in dem das ungesättigte Monomer der Formel (I) ein Propyltrialkoxysilylmethacrylatester ist.

7. Nagellack gemäß einem jeden der Patentansprüche 1 bis 6, in dem das Methacrylat-Monomer der Formel (II) das Methyl-, Ethyl-, Butyl-, tert.-Butyl-, Hexyl-, Dodecyl- oder Octadecyl-Methacrylat oder 2-N,N-Dimethylaminoethylmethacrylat ist.

8. Nagellack gemäß Patentanspruch 1, in dem das Acrylat der Formel (III) das Methyl-, Ethyl-, Butyl-, 2-Ethylhexyl-, Dodecyl- oder Octadecyl-Acrylat ist.

9. Nagellack gemäß einem jeden der Patentansprüche 1 bis 8, in dem das Copolymer mit einem Quaternisierungsmittel, ausgewählt aus Dimethylsulfat oder Ethylbromid, quaternisiert ist.

10. Nagellackzusammensetzung gemäß einem jeden der Patentansprüche 1 bis 9, wobei das organische Lösungsmittelsystem aus Aceton, Ethylacetat, Butylacetat, Methylethylketon, Methylacetat oder Mischungen derselben besteht.

11. Nagellackzusammensetzung gemäß einem jeden der Patentansprüche 1 bis 10, zusätzlich einen Weichmacher enthaltend.

12. Nagellackzusammensetzung gemäß einem jeden der Patentansprüche 1 bis 11, wobei der Weichmacher Benzylbenzoat oder Tributylphosphat ist.

13. Nagellackzusammensetzung gemäß einem jeden der Patentansprüche 1 bis 12, zusätzlich ein Farbmittel enthaltend.

## Revendications

1. Composition de vernis pour les ongles comprenant un agent filmogène et un système de solvant organique, ledit agent filmogène comprenant un copolymère résultant de la copolymérisation de:
(1) 1 à 30% en poids d'au moins un monomère de siloxanylalkylester de formule:

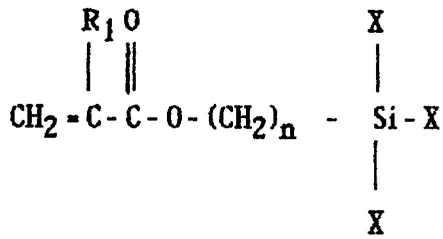

(I)

dans laquelle chaque X est $OCH_3$, $OCH_2CH_3$, $OCH_2CH_2CH_3$ ou un groupe phényle; $R_1$ est méthyle ou hydrogène; et n est un entier de 3 à 18;
(2) 10 à 90% en poids d'au moins un monomère de méthacrylate de formule:

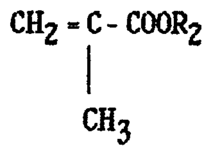

(II)

dans laquelle $R_2$ représente un radical alkyle linéaire ou ramifié ayant jusqu'à 18 atomes de carbone, ou le radical

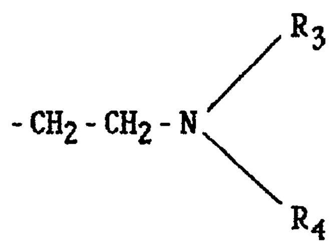

dans lequel $R_3$ et $R_4$ représentent un groupe alkyle ou hydroxyalkyle ayant de 1 à 4 atomes de carbone; et
(3) 1–90% en poids d'au moins un monomère d'acrylate de formule:

$$CH_2 = CH - COOR_5 \qquad (III)$$

dans laquelle $R_5$ représente un radical alkyle linéaire ou ramifié ayant jusqu'à 18 atomes de carbone, ou un radical aralkyle ayant de 7 à 16 atomes de carbone, ou un groupe hétéroalkyle ayant de 1 à 18 atomes de carbone dans lequel la chaîne carbonée est interrompue par un atome de O, N ou S.
2. Vernis pour les ongles selon la revendication 1 dans lequel ledit copolymère comprend:
(1) 5 à 20% en poids d'au moins un monomère de siloxanylalkylester de formule (I),
(2) 5 à 85% en poids d'au moins un monomère de méthacrylate de formule (II), et
(3) 5 à 85% en poids d'au moins un monomère d'acrylate de formule (III).
3. Vernis pour les ongles selon la revendication 1 ou 2 dans lequel ledit copolymère contient aussi au moins un autre monomère qui est la N-vinylpyrrolidone ou un acrylamide ou méthacrylamide de formule:

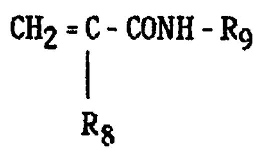

(IV)

dans laquelle $R_8$ représente H ou $-CH_3$ et $R_9$ représente H, $-CH_2OH$

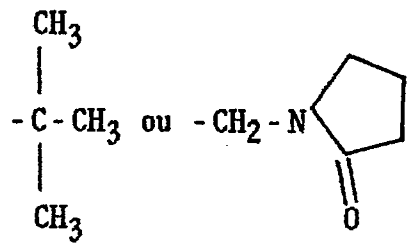

en une quantité de 1 à 20% en poids par rapport au poids total des monomères.

4. Vernis pour les ongles selon la revendication 3 dans lequel ledit autre monomère comprend de 2 à 15% en poids par rapport au poids total des monomères.

5. Vernis pour les ongles selon la revendication 3 ou 4 dans lequel l'acrylamide ou le méthacrylamide de formule (IV) est l'acrylamide, le méthacrylamide, le N-tert.-butylacrylamide, le N-hydroxyéthylméthacrylamide, le N-[(N'-pyrrolidone-2)-méthyl]acrylamide ou le n-hydroxyméthylacrylamide.

6. Vernis pour les ongles selon l'une des revendications 1 à 5 dans lequel le monomère insaturé de formule (I) est un ester de propyltrialcoxysilylméthacrylate.

7. Vernis pour les ongles selon l'une des revendications 1 à 6 dans lequel le monomère de méthacrylate de formule (II) est le méthyl-, éthyl-, butyl-, tertiobutyl-, hexyl-, dodécyl- ou octadécylméthacrylate ou le 2-N,N-diméthylaminoéthylméthacrylate.

8. Vernis pour les ongles selon la revendication 1 dans lequel l'acrylate de formule (III) est l'acrylate de méthyle, éthyle, butyle, 2-éthylhexyle, dodécyle ou d'octadécyle.

9. Vernis pour les ongles selon l'une des revendications 1 à 8 dans lequel le copolymère est quaternisé avec un agent de quaternisation choisi parmi le sulfate de diméthyle et le bromure d'éthyle.

10. Composition de vernis pour les ongles selon l'une des revendications 1 à 9 dans laquelle le système de solvant organique est l'acétone, l'acétate d'éthyle, l'acétate de butyle, la méthyléthylcétone, l'acétate de méthyle ou de mélanges de ceux-ci.

11. Composition de vernis pour les ongles selon l'une des revendications 1 à 10 comprenant en outre un plastifiant.

12. Composition de vernis pour les ongles selon la revendication 11 dans laquelle le plastifiant est le benzylbenzoate ou le tributylphosphate.

13. Composition de vernis pour les ongles selon l'une des revendications 1 à 12 comprenant en outre un colorant.